# EUROPEAN PATENT APPLICATION

(11) **EP 4 459 291 A1**
(43) Date of publication of application: **06.11.2024**
(21) Application number: 22915930.6
(22) Date of filing: 22.12.2022
(51) Int. Cl.: G01N 35/02, G01N 33/49, G01N 33/66, G01N 33/68, G01N 33/72

(54) **METHOD FOR MEASURING ELEMENT OF HEMOCYTE COMPONENT AND ELEMENT OF NON-HEMOCYTE COMPONENT IN MINUTE AMOUNT OF BLOOD, DEVICE, AND PIPETTE CARTRIDGE**

(30) Priority: 29.12.2021 JP 2021215357
(71) Applicant: Provigate Inc., Tokyo 113-0033 (JP)
(72) Inventor: KATAYAMA Norikazu, Tokyo 113-0033 (JP)
(74) Representative: Michalski Hüttermann & Partner Patentanwälte mbB
(86) International application number: PCT/JP2022/047497
(87) International publication number: WO 2023/127707

(57) **Abstract**

The present disclosure provides a method for measuring an element of a blood cell component and an element of a non-blood cell component in a minute amount of blood, including: providing a minute amount of blood; obtaining a first blood portion and a second blood portion from the minute amount of blood; measuring a first element related to a non-blood cell component in the first blood portion; hemolyzing the second blood portion; and measuring a second element related to a blood cell component in the second blood portion after the hemolysis.

## Description

### TECHNICAL FIELD

The present disclosure relates to a method for measuring an element of a blood cell component and an element of a non-blood cell component in a minute amount of blood.

### BACKGROUND ART

A blood cell component and a serum or plasma component in blood each contain useful biomarkers. Therefore, it is useful to perform multiple measurements on the two components from the same whole blood sample of a target subject.

For example, blood glucose, HbA1c, glycoalbumin (GA), etc. are measured for diabetes-related tests. HbA1c is generally measured by a colorimetric method or the like using a hemolyzed blood sample. On the other hand, blood glucose and glycoalbumin are likewise measured by a colorimetric method or the like, using serum or plasma after blood cell separation. The absorption wavelength of hemoglobin, which is a red pigment protein contained in a hemolyzed sample, affects the optical measurement of glucose and glycoalbumin using serum or plasma. Therefore, in order to simultaneously perform measurement of a substance(s) such as HbA1c, contained in a blood cell component, and measurement of a substance(s) such as blood glucose and glycoalbumin, contained in a serum component, it has been necessary to prepare a whole blood sample in such as to be capable of stably separating blood cells and serum/plasma. For example, if venous blood can be collected, it is possible to prepare a predetermined amount of whole blood sample.

### SUMMARY OF INVENTION

While there has recently been a demand for a faster, simpler, and less invasive blood test, simultaneous testing of a plurality of biomarkers, contained in a blood cell component and in a serum/plasma component, using a smaller amount of blood, e.g. capillary blood such as fingertip blood, has not yet been achieved because of the difficulty in stably separating the blood cell component and the serum/plasma component.

An embodiment of the present disclosure provides a technique for measuring a plurality of biomarkers using a small amount of blood. According to an embodiment of the present disclosure, there is provided a method for measuring an element of a blood cell component and an element of a non-blood cell component in a minute amount of blood, comprising: measuring a first element related to a non-blood cell component in a first blood portion of a minute amount of blood; hemolyzing a second blood portion of the minute amount of blood; and measuring a second element related to a blood cell component in the second blood portion after the hemolysis.

### BRIEF DESCRIPTION OF DRAWINGS

[FIG. 1] FIG. 1 is a flowchart of a method for measuring elements in a minute amount of blood according to an embodiment.
[FIG. 2] FIG. 2 is a flowchart of a method for measuring elements in a minute amount of blood according to an embodiment.
[FIG. 3] FIG. 3 is a block diagram of an apparatus for measuring elements in a minute amount of blood according to an embodiment.
[FIGS. 4A-4C] FIGS. 4A through 4C are front views illustrating the construction and operating procedure of a pipette cartridge for measuring elements in a minute amount of blood, according to an embodiment.
[FIGS. 4D-4F] FIGS. 4D through 4F are front views illustrating the construction and operating procedure of the pipette cartridge for measuring elements in a minute amount of blood, according to the embodiment.
[FIGS. 4G-4I] FIGS. 4G through 4I are front views illustrating the construction and operating procedure of the pipette cartridge for measuring elements in a minute amount of blood, according to the embodiment.
[FIG. 5A] FIG. 5A is a graph showing a correlation in GA % between the filtered serum and the venous serum obtained in Example 1.
[FIG. 5B] FIG. 5B is a graph showing a correlation in HbA1c % between the hemolyzed sample and the venous blood sample obtained in Example 1.
[FIG. 6] FIG. 6 is a graph showing a correlation in HbA1c % between the hemolyzed sample and the venous blood sample obtained in Example 2.
[FIG. 7] FIG. 7 is a graph showing a correlation in glucose concentration between the filtered serum and the venous serum obtained in Example 3.
[FIG. 8] FIG. 8 is a graph showing a correlation in 1,5-AG concentration between the filtered serum and the venous serum obtained in Example 4.
[FIG. 9] FIG. 9 is a graph showing a correlation in GA % between the filtered serum and the venous serum obtained in Example 5.

### DESCRIPTION OF EMBODIMENTS

### <1. Measurement Method>

An embodiment of the present disclosure provides a technique for measuring a plurality of biomarkers using a small amount of blood. According to an embodiment of the present disclosure, there is provided a method for measuring an element of a blood cell component and an element of a non-blood cell component in a minute amount of blood, comprising:
obtaining a first blood portion and a second blood portion from a minute amount of blood;
measuring a first element related to a non-blood cell component in the first blood portion;
hemolyzing the second blood portion;
and measuring a second element related to a blood cell component in the second blood portion after the hemolysis.

Such measurement of the elements using a minute amount of blood makes it possible, for example and without limitation, to test a plurality of biomarkers using a small amount of blood as point-of-care testing, or at an individual clinic or at home, or to easily perform health management.

As used herein, "a minute amount of blood" refers to such an amount that it is generally substantially impossible or substantially meaningless in the medical or industrial sense to perform a desired test or measurement by carrying out a process, including centrifugation, on that amount of blood.

The minute amount of blood may be equal to or smaller than 0.5 µL, 0.6 µL, 0.7 µL, 0.8 µL, 0.9 µL, 1 µL, 2 µL, 3 µL, 4 µL, 5 µL, 6 µL, 7 µL, 8 µL, 9 µL, 10 µL, 10 µL, 20 µL, 30 µL, 40 µL, or 50 µL.

In some embodiments, the minute amount of blood may be capillary blood or be derived therefrom. In some embodiments, the minute amount of blood may be fingertip blood. As used herein, "fingertip blood" generally refers to a droplet of capillary blood which has been formed on the body surface using a device such as a lancet. The blood can be collected using a capillary tube. Capillary blood may be likewise formed and collected on an earlobe, an upper arm, the abdomen, or the bottom of the foot (e.g., a newborn infant).

In some embodiments, the minute amount of blood may be venous blood or arterial blood, or be derived therefrom. In general, venous blood or arterial blood can be collected in a relatively large amount. However, in some cases, such blood can only be collected in a small amount.

In some embodiments, the minute amount of blood may be diluted. In some embodiments, the present method may include diluting the minute amount of blood. The minute amount of blood as neat blood may be diluted 2-fold, 3-fold, 4-fold, 5-fold, 6-fold, 7-fold, 8-fold, 9-fold, 10-fold, 15-fold, 20-fold, 25-fold, 30-fold, 40-fold, 50-fold, 60-fold, 70-fold, 80-fold, 90-fold, or 100-fold. As used herein, "a diluted liquid of a minute amount of blood" or a similar term may be used for the same meaning as "a minute amount of blood" unless there is a contradiction.

Dilution of the minute amount of blood may be performed by adding or mixing a predetermined diluent solution to or with the minute amount of blood. In some embodiments, the diluent solution does not substantially cause hemolysis. The diluent solution may be a saline solution. In some embodiments, the diluent solution is preferably an isotonic solution. The diluent solution may be a buffer solution such as a Good's buffer (e.g. HEPES).

In some embodiments, the first blood portion and the second blood portion may be obtained from the minute amount of blood. The minute amount of blood may be separated into the first blood portion and the second blood portion. The minute amount of blood may be separated into two portions. There may be some residue.

In some embodiments, the hemolysis may be performed using a hemolytic agent. As used herein, the term "hemolytic agent" refers to any substance that can lyse red blood cells in blood. The hemolytic agent may be a hypotonic solution. The hemolytic agent may be, for example and without limitation, pure water or a solution.

In some embodiments, the hemolytic agent may include cyanide ions (a solution of a cyanide or a cyano compound). Cyanide ions in such a hemolytic agent bind to released hemoglobin to form cyanmethemoglobin. The concentration of hemoglobin can be determined by measuring the amount of the cyanmethemoglobin using an absorbance method.

In some embodiments, the hemolytic agent may contain a surfactant. The surfactant in the hemolytic agent changes the steric structure of released hemoglobin. The concentration of hemoglobin can be determined by measuring the absorbance of the changed hemoglobin.

In some embodiments, the hemolytic agent may contain a staining solution which stains an arbitrary target substance. The staining solution in the hemolytic agent binds to a component (a target substance such as a protein, an antibody, an antigen, or a low-molecular substance) derived from lysed cells or cells other than lysed cells. The concentration of the component can be determined by optical measurement.

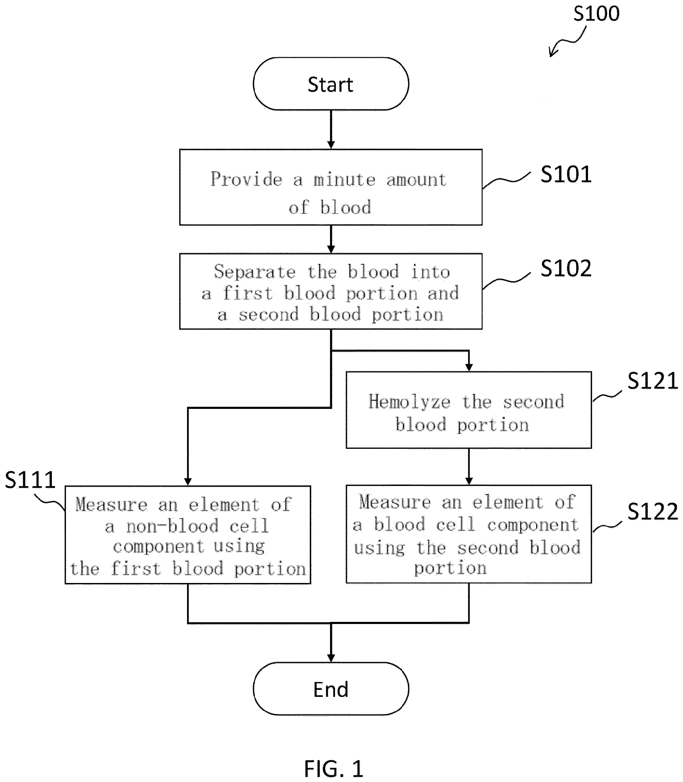

FIG. 1 shows a flowchart S100 of a measurement method according to an embodiment. A minute amount of blood is provided (S101). The minute amount of blood is separated into a first blood portion and a second blood portion. Alternatively, the first blood portion and the second blood portion may be taken from the minute amount of blood (S102). One or more elements of a non-blood cell component are measured using the first blood portion (S111). On the other hand, the second blood portion is hemolyzed (S121). Using the hemolyzed second blood portion, one or more elements of a blood cell component are measured (S122).

### <Volumetric Separation>

In the step of separating the minute amount of blood into the first blood portion and the second blood portion (S102), the minute amount of blood may be dispensed volumetrically. In some embodiments, the first blood portion and the second blood portion may be dispensed from the minute amount of blood by pipetting, pipette operation or by an automated dispenser. Alternatively, a portion of the minute amount of blood may be dispensed as the second blood portion (or the first blood portion), and the other portion may be used as the first blood portion (or the second blood portion).

In some embodiments, the present method may include passing the minute amount of blood through a serum separation filter. The first blood portion may be defined as the portion that has passed through the serum separation filter. The second blood portion may be defined as the portion that remains or has been captured in the serum separation filter. The second blood portion, captured in the filter, may include a blood cell component.

### <Separation Using Filter>

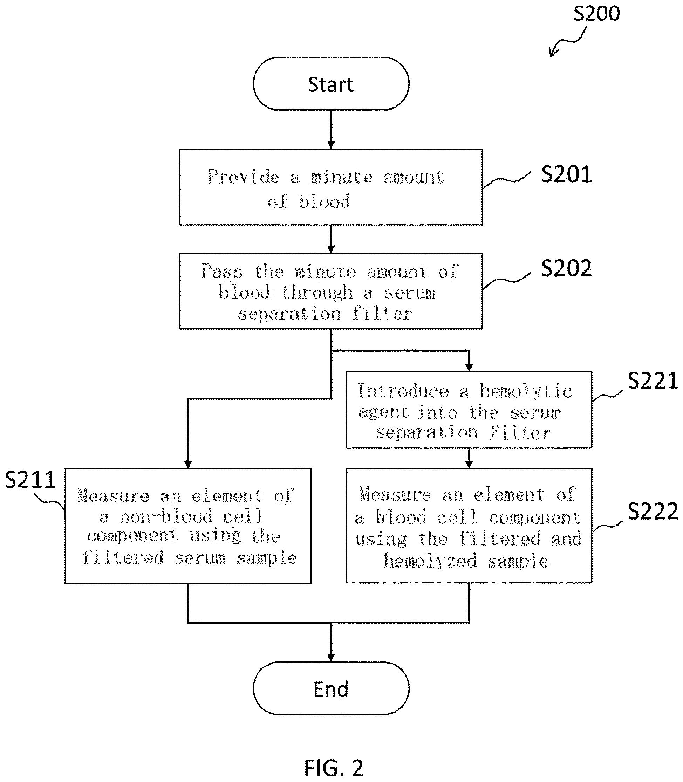

FIG. 2 shows a flowchart S200 of a measurement method according to an embodiment which uses a serum separation filter.

A minute amount of blood is provided (S201).

The minute amount of blood is passed through a serum separation filter (S202). The serum separation filter captures a blood cell component. The portion that has passed through the serum separation filter does not substantially contain a blood cell component, i.e., it is substantially composed of a non-blood cell component. A liquid component will remain in the serum separation filter; therefore, some non-blood cell component may remain in the filter. The blood portion (non-blood cell component, first blood cell component) that does not contain a blood cell component can thus be separated from the blood cell component (second blood cell component) by using the serum separation filter, and the both components can be collected.

An element(s) of the non-blood cell component is measured using the non-blood cell component (exemplarily referred to as "first blood portion" or "filtered serum sample") that has passed through the filter (S211).

On the other hand, a hemolytic agent is introduced into the serum separation filter (S221). The blood cell component captured in the serum separation filter comes into contact with the hemolytic agent and is hemolyzed.

Using the portion (second blood portion) consisting of the hemolyzed blood cell component, one or more elements of the blood cell component are measured (S222).

In some embodiments, the hemolytic agent may be introduced into the serum separation filter that has captured the second blood portion. In some embodiments, the hemolytic agent may be brought into contact with the second blood portion captured in the serum separation filter. The second blood portion may be hemolyzed in this manner.

As used herein, "serum separation" is used interchangeably with "blood separation," "plasma separation," "blood cell separation," and "plasma or serum separation." As used herein, "filter" is generally used interchangeably with "membrane", "film", or a laminate thereof. For example, "serum separation filter" has the same meaning as "blood cell separation membrane" unless otherwise specified. A blood cell separation filter is a material or member which can capture a blood cell component and discharge a non-blood cell component such as serum to the outside. A blood cell separation filter may have the ability to capture at least red blood cells. The serum separation filter may have the ability to capture cellular components (red blood cells, white blood cells, platelets).

The serum separation filter may be used for hemolysis also when the minute amount of blood is volumetrically separated into the first blood portion and the second blood portion. In some embodiments, the second blood portion obtained by pipetting may be passed through the serum separation filter. A hemolytic agent may be introduced into the serum separation filter that has captured the second blood portion. In some embodiments, the hemolytic agent may be brought into contact with the second blood portion captured in the serum separation filter. The second blood portion may be hemolyzed in this manner.

### <Measurement Target>

Measurement targets include an element(s) related to a blood cell component and an element(s) related to a non-blood cell component. Such measurement targets may include substances that are currently known or later discovered. The elements and substances shown in the present disclosure are presented by way of example only, and should not be construed as limiting in any way.

The method of the present disclosure may further comprise determining the concentration of a measurement target or a related value. For example, the albumin concentration, glycoalbumin concentration, HbA1c concentration, hemoglobin concentration, glucose concentration, and 1,5-AG concentration in a sample or solution may be determined. For example, a proportion (GA value, GA %) may be determined from the total albumin (ALB) concentration and the glycated albumin (GA) concentration. For example, a proportion (HbAlc value, HbA1c %) may be determined from the total hemoglobin (Hb) concentration and the glycated hemoglobin (HbA1c) concentration.

The element(s) (first element) to be measured in relation to a non-blood cell component may be selected from the group consisting of GA, glucose, and 1,5-AG. The element(s) (first element) to be measured in relation to a non-blood cell component may include or may be selected from, for example and without limitation, the following substances:
biochemical test-related substances such as total protein (TP), albumin (ALB), AST, ALT, γ-glutamyl transpeptidase (γ-GT), urea nitrogen (BUN), creatinine (CRE), creatine kinase (CK), CK-MB activity, amylase (AMY), pancreatic lipase (LIP), uric acid (UA), total cholesterol (T-CHO), triglycerides (TG), HDL cholesterol (HDL-C), LDL cholesterol (LDL-C), iron (Fe), and zinc (Zn);
vitamin-related substances;
electrolyte/blood gas-related substances such as calcium, potassium, and magnesium;
biochrome-related substances such as total bilirubin;
toxic substances/occupational medical metabolic substances;
antibiotics, antiepileptic drugs, immunosuppressants, cardiovascular drugs, psychoneurotic agents, and other drugs;
coagulation/fibrinolysis test-related substances such as fibrinogen (FIB) and D-dimer, hormones such as thyroid stimulating hormone (TSH), adrenocorticotropic hormone (ACTH), cortisol, adrenal medullary hormone, gonadal hormone, and insulin, and binding proteins;
endocrinological substances such as human brain natriuretic peptide (BNP), and human brain natriuretic peptide precursor N-terminal fragment (NT-proBNP);
tumor marker-related substances such as prostate specific antigen (PSA);
inflammation-related substances such as CRP;
hepatitis virus (type B, type C), human immunodeficiency virus (HIV), and various other viruses; infection-related substances such as Candida antibodies;
autoimmune test-related substances;
immunohematological test-related substances;
various immunoglobulins;
various cytokines;
allergy test-related substances;
various amino acids;
autoantibody test-related substances; and
insulin, ketone bodies, 3-hydroxybutyric acid, lactic acid, oxidized albumin, reduced albumin, etc.

The element(s) (second element) to be measured in relation to a blood cell component may be HbA1c. The second element(s) may be, for example and without limitation, various proteins, antigens, antibodies and low-molecular substances, derived from red blood cells. The element(s) (second element) to be measured in relation to a blood cell component may be, for example and without limitation, CRP (C-reactive protein), red blood cell creatine, glucose-6-phosphate (G6P), glutathione (GSH), and pyruvate kinase (PK).

### <2. Measurement System>

The present disclosure provides an apparatus, device or system for carrying out the measurement method disclosed herein. In some embodiments, a flow path device is provided. In some embodiments, a pipette cartridge for use in an automated analyzer is provided. The apparatus, etc. of the present disclosure are not limited to them; other ones may be possible.

### <Embodiment 1: Flow Path Device>

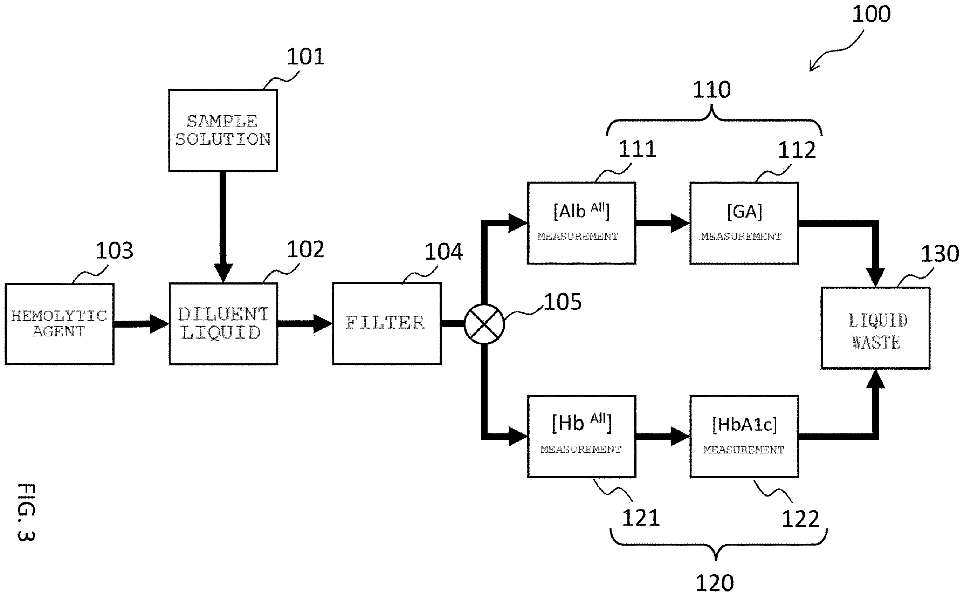

FIG. 3 shows a block diagram of an apparatus or device 100 which can measure both GA % and HbA1c %. Such an apparatus may be a stationary apparatus, a desktop apparatus, a handheld apparatus, or a flow path device.

The device 100 includes a diluent tank 102, a filter section 104, a first blood portion measuring section 110, and a second blood portion measuring section 120. The device 100 includes a valve 105 for switching the flow path between the first blood portion measuring section 110 and the second blood portion measuring section 120 after the filter section 104.

The device 100 shown in FIG. 3 includes a hemolytic agent tank 103 containing a hemolytic agent. In FIG. 3, the hemolytic agent tank 103 is disposed upstream of the diluent tank 102; however, it may be disposed at a different position.

The device 100 shown in FIG. 3 further includes a waste tank 130 that collects liquids discharged from the first blood portion measuring section 110 and the second blood portion measuring section 120. This allows for safe waste disposal.

The first blood portion measuring section 110 is configured to measure GA %. The first blood portion measuring section 110 includes a total ALB measuring section 111 for measuring the amount of total albumin, and a GA measuring section 112. These sections may be arranged fluidically in series or in parallel.

The second blood portion measuring section 120 is configured to measure HbA1c %. The second blood portion measuring section 120 includes a total Hb measuring section 121 for measuring the amount of total hemoglobin, and an HbA1c measuring section 122. These sections may be arranged fluidically in series or in parallel.

Blood as a sample solution is introduced from a sample solution device 101 into the diluent tank 102, and passes through the filter section 104. The valve 105 guides the filtered first blood portion (non-blood cell component) to the first blood portion measuring section 110. The total albumin amount and the GA amount in the non-blood cell component are measured by the total ALB measuring section 111 and the GA measuring section 112, respectively. The GA % is determined by dividing the measured GA amount by the measured total albumin amount.

Subsequently, the device 100 introduces the hemolytic agent from the hemolytic agent tank 103 into the filter section 104. The blood cell component captured by the filter section 104 is hemolyzed. At this stage, the valve 105 guides the second blood portion (blood cell component), hemolyzed in the filter section 104, to the second blood portion measuring section 120. The total hemoglobin amount and the HbA1c amount in the blood cell component are measured by the total Hb measuring section 121 and the HbA1c measuring section 122, respectively. The HbA1c % is determined by dividing the measured HbA1c amount by the measured total Hb amount.

The device 100 may include a liquid transfer system which can perform or control transfer of the above liquids.

### <Embodiment 2: Automated Dispenser>

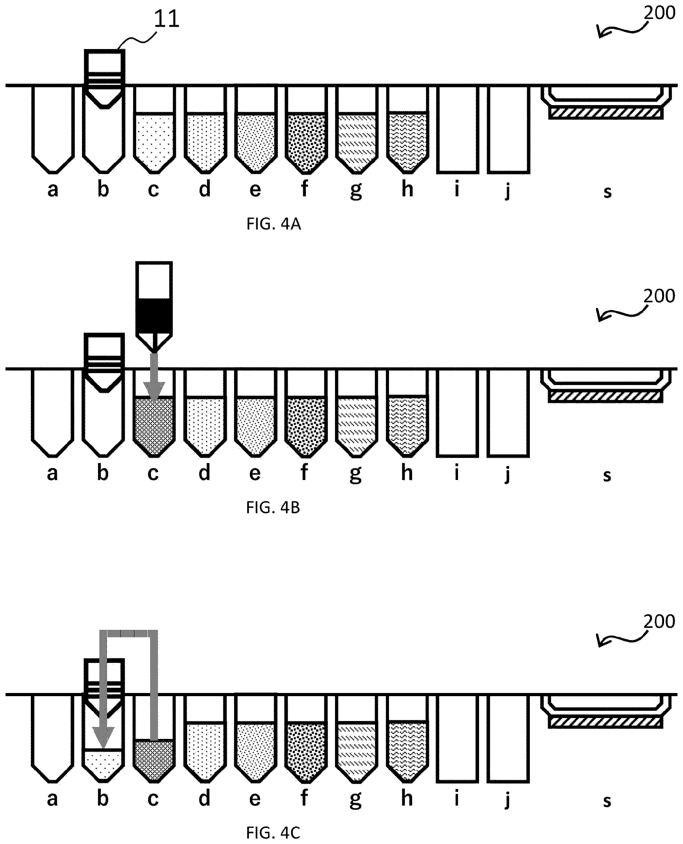

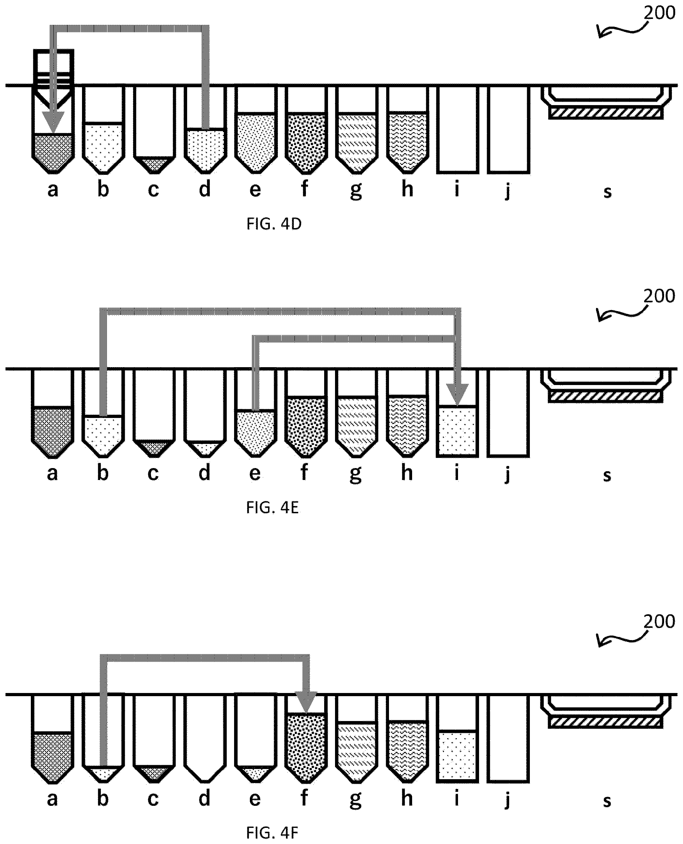

FIG. 4A shows a pipette cartridge 200 according to an embodiment. The pipette cartridge 200 includes wells a to j and a GA sensor s, and can measure both a GA value and an HbA1c value.

The wells a and b are initially empty. A serum separation filter (hereinafter referred to simply as a filter) 11 is removably mounted on the well b. The wells c to h contain (c) a diluent, (d) purified water as a hemolytic agent, (e) a BCP solution, (f) a protease solution, (g) a mixed solution of a denaturant for Hb, a color former for HbA1c measurement, and a protease, and (h) an HbA1c measurement reagent containing an oxidase (FPOX) and a peroxidase (POD), respectively. The wells i and j are (i) a well for optical measurement of albumin and (j) a well for optical measurement of HbA1c. An optical measurement system is installed in each of the wells i and j, so that the difference in intensity between incident light and output light can be measured as an absorbance.

The GA sensor s includes a hydrogen peroxide electrode on which a fructosyl amino acid oxidase (FAOD) membrane is formed. Peptide fragments react with FAOD to generate hydrogen peroxide. The hydrogen peroxide electrode can measure the generated hydrogen peroxide.

A measurement procedure will now be described using FIGS. 4B through 4I. It is to be noted that the construction of the pipette cartridge, the types, numbers and amounts of the solutions and reagents used, the measurement methods and procedures, etc. are presented by way of example only, and the present invention should not be construed as being limited thereto; other embodiments and aspects are possible.

First, a whole blood sample, either collected or provided, is added to the well c and mixed with the diluent. (FIG. 4B)

The diluted blood is passed through the filter 11. The non-blood cell component (plasma or serum) that has passed through the filter is collected in the well b, while the blood cell component is captured by the filter 11. (FIG. 4C)

The filter 11 is removed from the well b, and is mounted on the well a. The hemolytic agent is taken from the well d, and is added to the filter 11 mounted on the well a. The red blood cells captured in the filter 11 are hemolyzed, and the hemolyzed component is collected in the well a.

### (FIG. 4D)

A portion of the non-blood cell component is taken from the well b, and is placed in the well i for optical measurement of ALB. The BCP solution in the well e is placed in the well i and mix with the non-blood cell component. The BCP binds to albumin. An optical measurement is performed on the BCP to determine the albumin concentration of the sample. (FIG. 4E)

The remainder of the non-blood cell component is taken from the well b, placed in the well f, and mixed with the protease solution. Albumin is degraded by the protease. The digestion reaction is generally promoted by heating the solution in the well f. Peptide fragments of albumin are thereby produced. (FIG. 4F)

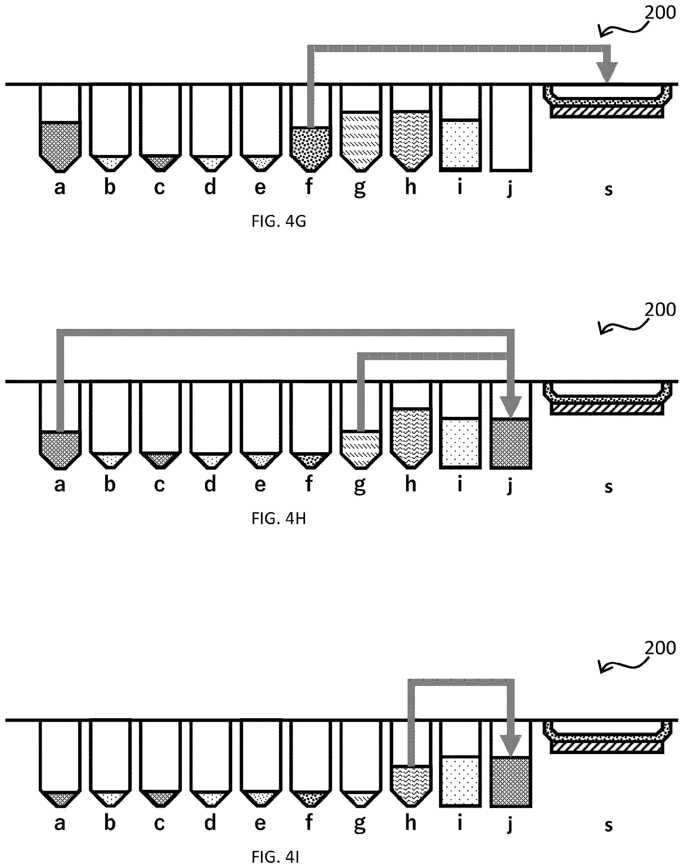

The solution containing the peptide fragments of albumin is taken from the well f, and is introduced into the sensor s. The peptide fragments react with the FAOD membrane to produce hydrogen peroxide. A redox reaction between the hydrogen peroxide and the electrode is converted into an electrical signal. The sensor s outputs the electrical signal. The GA concentration in the sample can be determined in this manner. (FIG. 4G)

The hemolyzed component is taken from the well a, and is placed in the optical measurement well j. The mixed solution of a denaturant for Hb, a color former for A1c measurement, and a protease is taken from the well g, placed in the optical measurement well j, and mixed with the hemolyzed component. The denaturant denatures hemoglobin in the hemolytic component. The total hemoglobin concentration in the sample can be determined by measuring the absorbance of the denatured hemoglobin. At the same time, the protease degrades the denatured hemoglobin to produce a glycated dipeptide. (FIG. 4H)

The HbA1c measurement reagent is taken from the well h, and is placed in the optical measurement well j. A glycated dipeptide reacts with the oxidase to generate hydrogen peroxide. The hydrogen peroxide produces methylene blue from the color former in the presence of POD. The concentration of glycated hemoglobin in the sample can be determined by measuring the color absorbance of the methylene blue. (FIG. 4I)

In some embodiments, an optical well may be provided instead of the sensor s, and the GA concentration may be optically measured using an optical reagent such as a coloring reagent.

### <3. Examples>

### <Example 1: Measurement of GA (%) and HbA1c (%) via Filtering>

Fingertip blood samples as measurement samples were collected from 11 healthy individuals. A blood droplet was formed on a fingertip using a lancet (Nipro LS Lancet, Nipro Corporation), and 10 µL of blood was collected from the droplet using a micropipette. 40 µL of a diluent buffer (10 mM HEPES, 150 mM NaCl, pH 8.0) was mixed with the collected blood to prepare 50 µL of diluted liquid.

The diluted liquid was passed through a blood separation filter, and a filtered fingertip blood serum sample was collected. Thereafter, 80 µL of ultrapure water as a hemolytic liquid was added to the blood separation filter. The hemolytic liquid that had passed through the blood separation filter exhibited a red color, which indicated hemolysis. The filtered and hemolyzed fingertip blood sample was collected.

GA (%) was measured for 2 µL of the filtered serum sample using a high-performance liquid chromatography (HPLC) apparatus (Shimadzu Corporation). On the other hand, HbA1c (%) was measured using an automated diabetes test item analyzer DM-JACK Ex+ (Minaris Medical Co., Ltd.) and an HbA1c measurement reagent (MetaboLead HbA1c, Minaris Medical Co., Ltd.) which uses an enzymatic method as a measurement principle.

As a comparative example, venous blood samples were collected from the same 11 healthy individuals, and each sample was centrifuged. Using the serum component of the centrifuged venous blood sample, GA (%) was measured in the same manner as that described above for fingertip blood. Further, HbA1c (%) was measured using the blood cell layer of the same centrifuged venous blood sample in the same manner as that described above for fingertip blood.

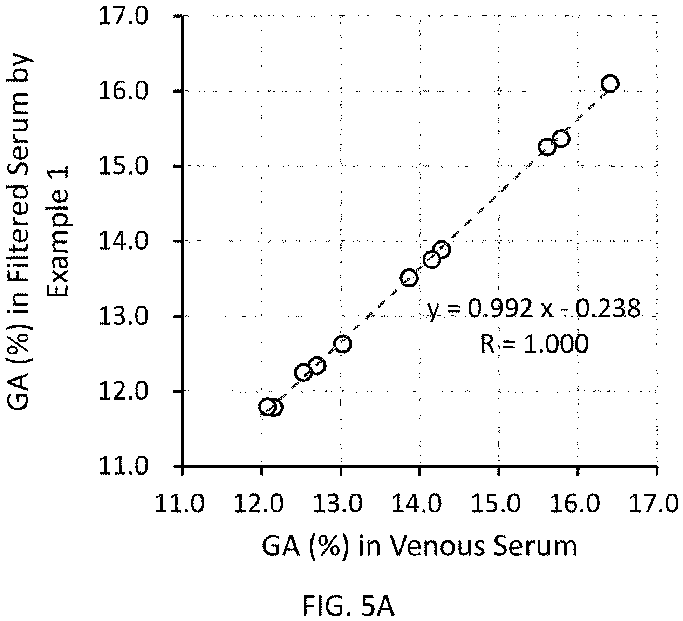

FIG. 5A shows the relationship between GA (%) in the filtered fingertip blood serum samples and GA (%) in the centrifuged venous blood serum samples. They have a very strong positive correlation (y = 0.992x - 0.238, correlation coefficient R = 1.000). The results suggest that the use of serum, obtained by filtering of a minute amount of blood, enables the same GA (%) measurement as that performed by the use of venous blood.

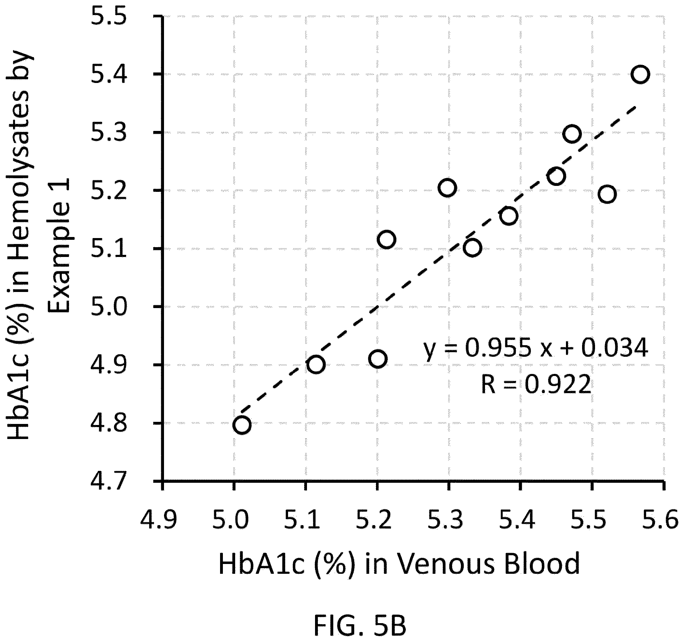

FIG. 5B shows the relationship between HbA1c (%) of the filtered and hemolyzed fingertip blood samples and HbA1c (%) of the venous blood samples. They have a very strong positive correlation (y = 0.955x + 0.034, correlation coefficient R = 0.922). The results suggest that the use of a hemolyzed blood sample derived from the blood cell component of a minute amount of blood, remaining in a blood separation filter after filtering of the blood, enables the same HbA1c (%) measurement as that performed by the use of venous blood.

Thus, the results revealed that both GA (%) and HbA1c (%) in blood can be determined from the same minute amount of blood likewise from venous blood.

### <Example 2: Measurement of GA (%) and HbA1c (%) Without Filtering>

Fingertip blood samples as measurement samples were collected from 12 healthy individuals. The fingertip blood samples were collected and a diluted liquid was prepared in the same manner as in Example 1.

26 µL of the diluted liquid was taken and passed through a blood separation filter, and a filtered fingertip blood serum sample was collected. 120 µL of a hemolytic agent (ultrapure water) was added to and mixed with 24 µL of the diluted liquid that had not been subjected to filtering. The resulting mixture exhibited a red color, which indicated hemolysis. The unfiltered hemolyzed fingertip blood sample was collected.

GA (%) and HbA1c (%) were measured in the same manner as in Example 1.

As a comparative example, venous blood samples were collected from the same 12 healthy individuals, and each sample was centrifuged. Using the centrifuged venous blood, GA (%) and HbA1c (%) were measured in the same manner.

GA (%) in the filtered fingertip blood serum samples and GA (%) in the centrifuged venous blood serum samples had a very strong positive correlation (not shown) similar to that shown in FIG. 5A.

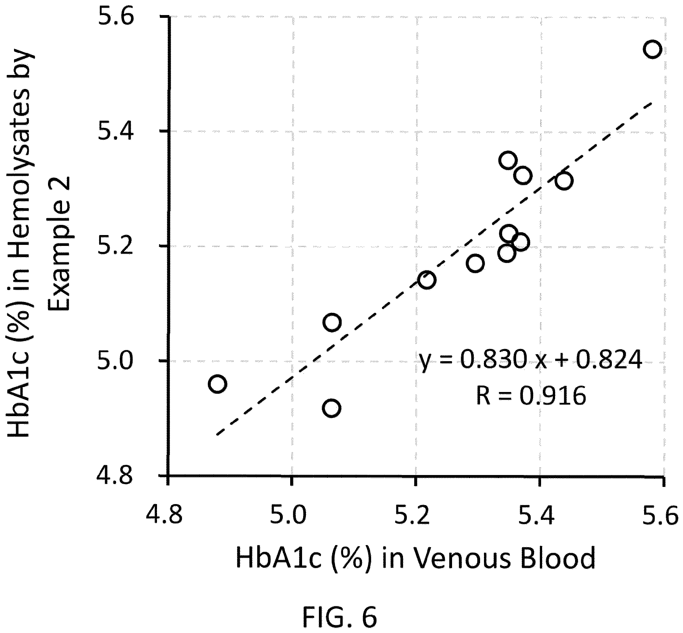

FIG. 6 shows the relationship between HbA1c (%) of the unfiltered hemolyzed fingertip blood samples and HbA1c (%) of the centrifuged venous blood samples. They have a very strong positive correlation (y = 0.830x + 0.824, R = 0.916). The results suggest that the use of an unfiltered hemolyzed blood sample, derived from the blood cell component of a minute amount of blood, enables the same HbA1c (%) measurement as that performed by the use of a hemolyzed blood sample derived from the blood cell component of a minute amount of blood, remaining in a blood separation filter after filtering of the blood.

### <Example 3: Measurement-1 of Other Biomarkers Using Filtered Serum Sample>

Measurement of blood glucose using a filtered serum sample was examined.

In the same manner as in Example 1, fingertip blood samples as measurement samples were collected from 10 healthy individuals, and a diluted liquid was prepared. Thereafter, a filtered fingertip blood serum sample was collected in the same manner as in Example 1.

Blood glucose was measured for 5 µL of the filtered serum sample using an automated diabetes test item analyzer DM-JACK Ex+ (Minaris Medical Co., Ltd.) and a glucose reagent (Determiner L GLU HK, Minaris Medical Co., Ltd.) which uses an enzymatic method as a measurement principle.

As a comparative example, venous blood samples were collected from the same 10 healthy individuals with the same timing as the collection of fingertip blood samples. After each sample was centrifuged, the serum was collected. Using the serum sample, blood glucose was measured in the same manner.

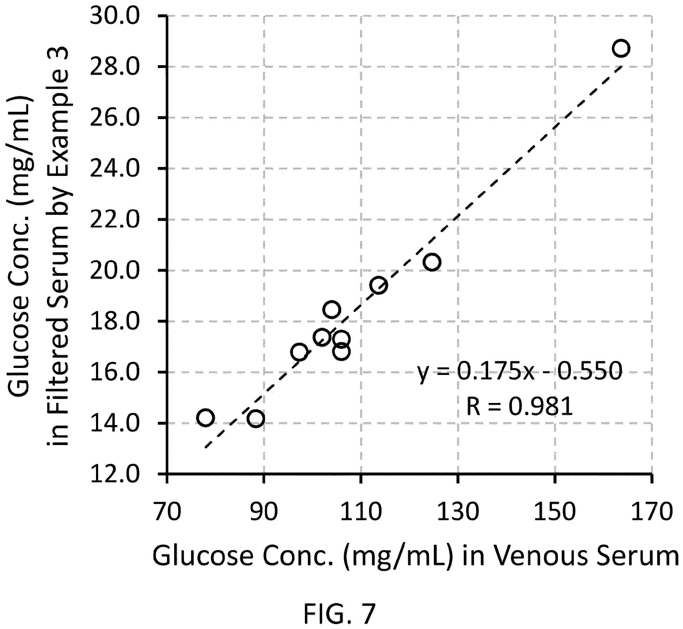

FIG. 7 shows the relationship between the blood glucose concentrations in the filtered fingertip blood serum samples and the blood glucose concentrations in the centrifuged venous blood serum samples. They have a very strong positive correlation (y = 0.175x - 0.550, correlation coefficient R = 0.981).

Thus, the data demonstrates that not only GA (%) but various other biomarkers, including blood glucose, can also be determined from a minute amount of blood.

### <Example 4: Measurement-2 of Other Biomarkers Using Filtered Serum Sample>

Measurement of 1,5-AG using a filtered serum sample was examined.

In the same manner as in Example 1, fingertip blood samples as measurement samples were collected from seven healthy individuals, and a diluted liquid was prepared. Thereafter, a filtered fingertip blood serum sample was collected in the same manner as in Example 1.

1,5-AG was measured for 8 µL of the filtered serum sample using an automated diabetes test item analyzer DM-JACK Ex+ (Minaris Medical Co., Ltd.) and a 1,5-AG measurement reagent (Determiner L 1,5-AG, Minaris Medical Co., Ltd.) which uses an enzymatic method as a measurement principle.

As a comparative example, venous bloods were collected from the same seven healthy individuals with the same timing as the collection of fingertip bloods. After each blood was centrifuged, the serum was collected. Using the serum sample, 1,5-AG was measured in the same manner.

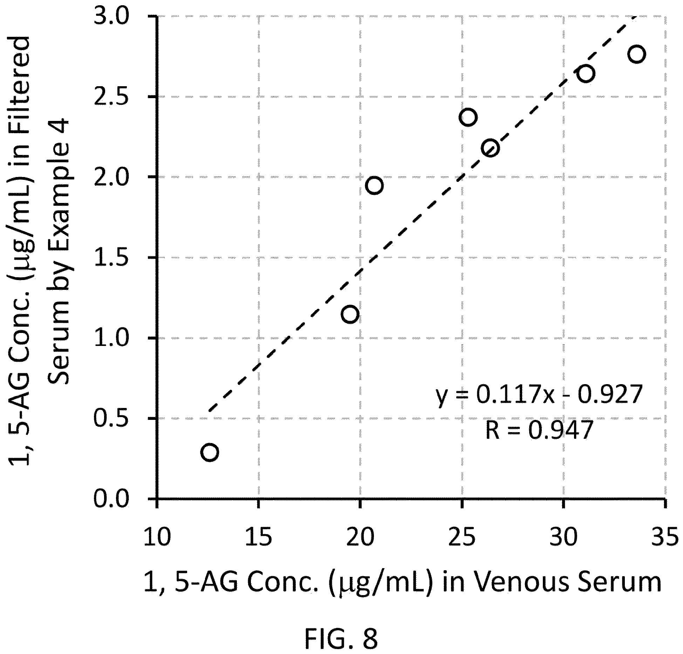

FIG. 8 shows the comparative results between the 1,5-AG concentrations in the filtered fingertip blood serum samples and the 1,5-AG concentrations in the centrifuged venous blood serum samples. They have a very strong positive correlation (y = 0.117x - 0.927, correlation coefficient R = 0.947).

Thus, the data demonstrates that not only GA (%) but various other biomarkers, including 1,5-AG, can also be determined from a minute amount of blood.

### <Example 5: Measurement of GA (%) and HbA1c (%); Examination of Different GA (%) Measurement Methods>

In the same manner as in Example 1, fingertip blood samples as measurement samples were collected from seven healthy individuals, and a diluted liquid was prepared. Thereafter, a filtered fingertip blood serum sample was collected in the same manner as in Example 1.

GA (%) was measured using an automated diabetes test item analyzer DM-JACK Ex+ (Minaris Medical Co., Ltd.) and a GA (%) measurement reagent (Lucica GA-L, Asahi Kasei Pharma Corporation) which uses an enzymatic method as a measurement principle.

As a comparative example, venous bloods were collected from the same seven healthy individuals, and each venous blood was centrifuged and the serum was collected. Using the serum sample (centrifuged venous blood serum sample), GA (%) was measured in the same manner.

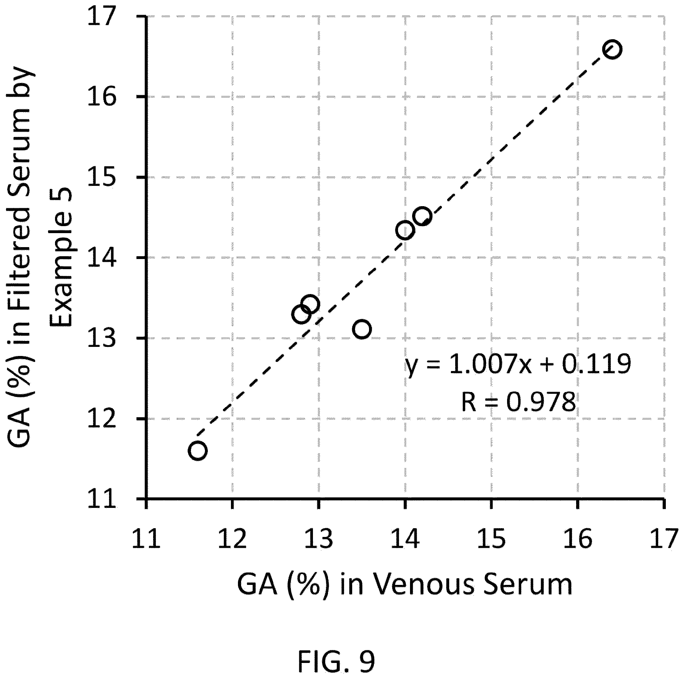

FIG. 9 shows the relationship between GA (%) of the filtered fingertip blood serum samples and GA (%) of the venous blood-derived serum samples. They have a very strong positive correlation (y = 1.007x + 0.119, correlation coefficient R = 0.978).

The results suggest that a measurement based on an enzymatic method can also be performed using a filtered fingertip blood serum sample, and that GA (%) and HbA1c (%) can be measured simultaneously by obtaining samples for the measurements.

The present disclosure includes the following embodiments:

### A001

A method for measuring an element of a blood cell component and an element of a non-blood cell component in a minute amount of blood, comprising:
providing a minute amount of blood;
obtaining a first blood portion and a second blood portion from the minute amount of blood;
measuring a first element related to a non-blood cell component in the first blood portion;
hemolyzing the second blood portion; and
measuring a second element related to a blood cell component in the second blood portion after the hemolysis.

### A002

The method according to A001,
wherein the step of providing a minute amount of blood includes diluting the minute amount of blood.

### A001b

A method for measuring an element of a blood cell component and an element of a non-blood cell component in a minute amount of blood, comprising:
diluting a minute amount of blood (providing a diluted liquid of a minute amount of blood);
obtaining a first blood portion (a first diluted liquid portion) and a second blood portion (a second diluted liquid portion) from the diluted liquid of the minute amount of blood;
measuring a first element related to a non-blood cell component in the first blood portion (the first diluted liquid portion);
hemolyzing the second blood portion (the second diluted liquid portion); and measuring a second element related to a blood cell component in the second blood portion (the second diluted liquid portion) after the hemolysis.

### A011

The method according to A001 or A002,
wherein the amount of the minute amount of blood is 50 µL or less.

### A012

The method according to A001 or A002,
wherein the amount of the minute amount of blood is 10 µL or less.

### A013

The method according to A001 or A002,
wherein the amount of the minute amount of blood is 5 µL or less.

### A021

The method according to any one of A001 to A012,
wherein the minute amount of blood is capillary blood.

### A022

The method according to any one of A001 to A021,
wherein the minute amount of blood is fingertip blood.

### A031

The method according to any one of A001 to A022,
wherein the step of obtaining a first blood portion and a second blood portion from the minute amount of blood comprises passing the minute amount of blood through a serum separation filter, and wherein the first blood portion is a portion that has passed through the serum separation filter, and the second blood portion is a portion that remains or has been captured in the serum separation filter.

### A032

The method according to A031,
wherein the step of hemolyzing the second blood portion comprises introducing a hemolytic agent into the serum separation filter that has captured the second blood portion, or bringing the second blood portion, captured in the serum separation filter, into contact with a hemolytic agent.

### A041

The method according to any one of A001 to A022,
wherein the step of obtaining a first blood portion and a second blood portion from the minute amount of blood
comprises dispensing a portion of the minute amount of blood as the second blood portion, and using the other portion as the first blood portion.

### A042

The method according to A0041,
wherein the step of hemolyzing the second blood portion comprises passing the second blood portion through a serum separation filter, and introducing a hemolytic agent into the serum separation filter that has captured the second blood portion.

### A051

The method according to any one of A001 to A042,
wherein the first element related to the non-blood cell component is a substance (a value for the substance) selected from the group consisting of GA, glucose, and 1,5-AG.

### A061

The method according to any one of A001 to A051,
wherein the second element related to the blood cell component is HbA1c (a value for the substance).

### B001

An apparatus or device for measuring an element of a blood cell component and an element of a non-blood cell component in a minute amount of blood, comprising:
a blood introduction section;
a filter having the ability to separate a blood cell component from blood that has been introduced into the filter;
a first blood portion measuring section for measuring an element of a non-blood cell component that has passed through the filter:
   a hemolytic agent tank which contains a hemolytic agent for hemolyzing a blood cell component that has been captured in the filter;
   a second blood portion measuring section for measuring an element of the blood cell component obtained by the hemolysis in the filter; and
   a liquid transfer system.

### B011

The apparatus or device according to B001,
further comprising a waste tank for receiving a liquid discharged from the first blood portion measuring section and/or the second blood portion measuring section.

### B021

The apparatus or device according to B001 or B011,
wherein the first blood portion measuring section is configured to measure at least one of GA%, a glucose value, and a 1,5-AG value,
and the second blood portion measuring section is configured to measure HbA1c %.

### B022

The apparatus or device according to any one of B001 to B021,
wherein the first blood portion measuring section is configured to measure GA%, and comprises a total albumin measuring section and a GA measuring section.

### B101

A pipette cartridge to be used in an automated dispenser to measure an element of a blood cell component and an element of a non-blood cell component in a minute amount of blood, comprising:
(a) a well which is configured to mount a filter thereon and which receives a first blood cell component (non-blood cell component);
(b) a well which is configured to mount the filter thereon and which receive a second blood cell component (blood cell component);
(c) a diluent well containing a diluent;
(d) a hemolytic agent storage well or hemolytic agent well containing a hemolytic agent;
(e/f) a first reagent well containing a reagent for measuring an element of the first blood cell component;
(g/h) a second reagent well containing a reagent for measuring an element of the second blood cell component;
(i) an optical measurement well for measuring the element of the first blood cell component; and
(j) an optical measurement well for measuring the element of the second blood cell component.

### B111

The pipette cartridge according to B101,
wherein the element of the non-blood cell component is a substance selected from the group consisting of GA, glucose, and 1,5-AG,
and the element of the blood cell component is HbA1c.

### B121

The pipette cartridge according to B111,
wherein the element of the non-blood cell component is GA.

### B122

The pipette cartridge according to B121,
wherein the first reagent well containing a reagent for measuring an element of the first blood cell component comprises
(e) a BCP solution well containing a BCP solution, and
(f) a protease solution well containing a protease solution, and wherein the second reagent well containing a reagent for measuring an element of the second blood cell component comprises
(g) a first HbA1c reagent well containing a mixed solution of a denaturant for Hb and a color former for HbA1c measurement, and
(h) a second HbA1c reagent well containing a protease, an oxidase (FPOX), and a peroxidase

### (POD).

### B131

The pipette cartridge according to any one of B101 to B122,
further comprising (s) an electrochemical sensor.

### B132

The pipette cartridge according to B 13 1,
wherein the electrochemical sensor (s) comprises a hydrogen peroxide electrode, and an FAOD membrane provided thereon.

While certain embodiments and examples of the present disclosure have been described, the embodiments and examples are presented merely to illustrate the present disclosure. For example, the embodiments have been described in detail to facilitate clear understanding of the present disclosure, and various changes and modifications may be made to dimensions, constructions, materials, and circuits as necessary. It is to be understood that embodiments, in which some of the above-described features of the present disclosure are combined in a desired manner, may fall within the scope of the present disclosure. The claims are intended to cover such variations to embodiments as fall within the scope of the technical concept of the present disclosure. Thus, the embodiments and examples disclosed herein are presented by way of illustration and should not be considered as limiting the scope of the present disclosure.

## Claims

1. A method for measuring an element of a blood cell component and an element of a non-blood cell component in a minute amount of blood, comprising:
providing a minute amount of blood;
obtaining a first blood portion and a second blood portion from the minute amount of blood;
measuring a first element related to a non-blood cell component in the first blood portion;
hemolyzing the second blood portion; and
measuring a second element related to a blood cell component in the second blood portion after the hemolysis.

2. The method according to claim 1,
wherein the step of providing a minute amount of blood includes diluting the minute amount of blood.

3. The method according to claim 1 or 2,
wherein the amount of the minute amount of blood is 10 µL or less.

4. The method according to claim 1 or 2,
wherein the amount of the minute amount of blood is 5 µL or less.

5. The method according to any one of claims 1 to 3,
wherein the minute amount of blood is capillary blood.

6. The method according to any one of claims 1 to 4,
wherein the minute amount of blood is fingertip blood.

7. The method according to any one of claims 1 to 6,
wherein the step of obtaining a first blood portion and a second blood portion from the minute amount of blood comprises passing the minute amount of blood through a serum separation filter,
and wherein the first blood portion is a portion that has passed through the serum separation filter,
and the second blood portion is a portion that remains or has been captured in the serum separation filter.

8. The method according to claim 7,
wherein the step of hemolyzing the second blood portion comprises introducing a hemolytic agent into the serum separation filter that has captured the second blood portion, or bringing the second blood portion, captured in the serum separation filter, into contact with a hemolytic agent.

9. The method according to any one of claims 1 to 6,
wherein the step of obtaining a first blood portion and a second blood portion from the minute amount of blood comprises
dispensing a portion of the minute amount of blood as the second blood portion, and
using the other portion as the first blood portion.

10. The method according to claim 9,
wherein the step of hemolyzing the second blood portion comprises passing the second blood portion through a serum separation filter, and introducing a hemolytic agent into the serum separation filter that has captured the second blood portion.

11. The method according to any one of claims 1 to 10,
wherein the first element related to the non-blood cell component is a substance selected from the group consisting of GA, glucose, and 1,5-AG.

12. The method according to any one of claims 1 to 11,
wherein the second element related to the blood cell component is HbA1c.

13. An apparatus for measuring an element of a blood cell component and an element of a non-blood cell component in a minute amount of blood, comprising:
a blood introduction section;
a filter having the ability to separate a blood cell component from blood that has been introduced into the filter;
a first blood portion measuring section for measuring an element of a non-blood cell component that has passed through the filter:
a hemolytic liquid tank which contains a hemolytic agent for hemolyzing a blood cell component that has been captured in the filter;
a second blood portion measuring section for measuring an element of the blood cell component obtained by the hemolysis in the filter; and
a liquid transfer system.

14. The apparatus according to claim 13,
further comprising a waste tank for receiving a liquid discharged from the first blood portion measuring section and/or the second blood portion measuring section.

15. The apparatus according to claim 13 or 14,
wherein the first blood portion measuring section is configured to measure at least one of GA%, a glucose concentration, and a 1,5-AG concentration,
and the second blood portion measuring section is configured to measure HbA1c %.

16. The apparatus or device according to any one of claims 13 to 15,
wherein the first blood portion measuring section is configured to measure GA%, and comprises a total albumin measuring section and a GA measuring section.

17. A pipette cartridge to be used in an automated dispenser to measure an element of a blood cell component and an element of a non-blood cell component in a minute amount of blood, comprising:
(a) a well which is configured to mount a filter thereon and which receives a first blood cell component (non-blood cell component);
(b) a well which is configured to mount the filter thereon and which receive a second blood cell component (blood cell component);
(c) a diluent well containing a diluent;
(d) a hemolytic agent storage well or hemolytic agent well containing a hemolytic agent;
(e/f) a first reagent well containing a reagent for measuring an element of the first blood cell component;
(g/h) a second reagent well containing a reagent for measuring an element of the second blood cell component;
(i) an optical measurement well for measuring the element of the first blood cell component; and
(j) an optical measurement well for measuring the element of the second blood cell component.

18. The pipette cartridge according to claim 17,
wherein the element of the non-blood cell component is a substance selected from the group consisting of GA, glucose, and 1,5-AG,
and the element of the blood cell component is HbA1c.

19. The pipette cartridge according to claim 18,
wherein the element of the non-blood cell component is GA.

20. The pipette cartridge according to claim 19,
wherein the first reagent well containing a reagent for measuring an element of the first blood cell component comprises
(e) a BCP solution well containing a BCP solution, and
(f) a protease solution well containing a protease solution,
and wherein the second reagent well containing a reagent for measuring an element of the second blood cell component comprises
(g) a first HbA1c reagent well containing a mixed solution of a denaturant for Hb and a color former for HbA1c measurement, and
(h) a second HbAlc reagent well containing a protease, an oxidase (FPOX), and a peroxidase (POD).

21. The pipette cartridge according to any one of claims 17 to 20,
further comprising (s) an electrochemical sensor.

22. The pipette cartridge according to claim 21,
wherein the electrochemical sensor (s) comprises a hydrogen peroxide electrode, and an FAOD membrane provided thereon.
